# EUROPÄISCHE PATENTSCHRIFT

(11) **EP 0 957 360 B1**
(45) Veröffentlichungstag und Bekanntmachung des Hinweises auf die Patenterteilung: **31.07.2002**
(21) Anmeldenummer: 99108860.0
(22) Anmeldetag: 04.05.1999
(51) Int. Cl.: G01N 33/53, G01N 33/576, G01N 33/569

(54) **Entstörung durch Rheumafaktoren**
Removal of disturbances by rheumatoid factors
Elimination de perturbanions par facteurs rhumatismaux

(30) Priorität: 06.05.1998 DE 19820239; 23.03.1999 DE 19913117
(43) Veröffentlichungstag der Anmeldung: 17.11.1999
(73) Patentinhaber: Roche Diagnostics GmbH, 68305 Mannheim (DE)
(72) Erfinder: Wehner, Rainer ,Dr., 82327 Tutzing (DE); Donie, Frederic Dr., 82377 Penzberg (DE); Ofenloch-Hähnle, Beatus, Dr., 82398 Polling (DE)
(74) Vertreter: Weiss, Wolfgang, Dipl.-Chem. Dr.

(56) Entgegenhaltungen:
- EP-A1- 0 483 512
- EP-A2- 0 617 285
- EP-A2- 0 809 111
- WO-A1-85/02258
- US-A- 5 358 852

## Beschreibung

Die Erfindung betrifft ein Verfahren zur Bestimmung eines Analyten, wobei zur Verringerung bzw. Vermeidung eines Hook-Effekts als Entstörungsreagenz Rheumafaktoren oder Rheumafaktor-ähnliche Substanzen zugesetzt werden. Weiterhin betrifft die Erfindung für die Durchführung des Verfahrens geeignete Reagenzienkits.

Zur quantitativen Bestimmung von Analyten in einer Probe werden häufig sog. Sandwich-Assays verwendet, wobei zwei gegen gleiche oder verschiedene Epitope des Analyten gerichtete Rezeptoren mit einer den zu bestimmenden Analyten enthaltenden Probe inkubiert werden. Dabei ist vorzugsweise ein erster löslicher Rezeptor direkt oder indirekt mit einem signalerzeugenden System, d.h. mit einer Markierung gekoppelt, während - in einem heterogenen Nachweisverfahren - ein zweiter Rezeptor an eine Festphase gekoppelt vorliegt oder mit einer Bindekomponente versehen ist, wie z.B. Biotin, die zur Bindung mit einer entsprechend beschichteten Festphase fähig ist.

Bei einer Anzahl diagnostisch wichtiger Parameter kann die Analytkonzentration in der Probe stark variieren, was bedeutet, daß ein breiter Meßbereich wünschenswert bzw. sogar erforderlich ist. Bei der Bestimmung derartiger Analyten ist es einerseits diagnostisch wichtig, einen möglichst exakten Wert in hohen Konzentrationsbereichen zu erhalten. Andererseits muß auch in unteren Konzentrationsbereichen eine exakte Bestimmung durchführbar sein, um zu einer qualitativ korrekten Diagnose Ja/Nein zu gelangen, die ihrerseits grundlegende therapeutische Konsequenzen nach sich ziehen kann.

Ein Problem bei hohen Analytkonzentrationen in einer zu untersuchenden Probe ist der sogenannte "High Dose Hook-Effekt", worunter ein Rückgang des Meßsignals bei sehr hohen Analytkonzentrationen zu verstehen ist. In diesem Fall kann ein bestimmtes Meßsignal durch zwei unterschiedliche Analytkonzentrationen hervorgerufen werden. Dieser "Hook-Effekt" schränkt die Anwendung von Sandwich-Assays erheblich ein.

Besonders gravierend ist das Auftreten des Hook-Effekts bei Einschritt-Sandwich-Assays. Als Einschritt-Assay werden alle Tests bezeichnet, bei denen der zu bestimmende Analyt mit mindestens zwei für ihn spezifischen, d.h. mit ihm ein "Sandwich" bildenden Rezeptoren in derselben Lösung zur Reaktion gebracht und dabei z.B. durch Immobilisierung an einer Festphase nachgewiesen wird, im Gegensatz zur sequentiellen Reaktionsführung, bei der nach Reaktion des Analyten mit einem ersten spezifischen Rezeptor und Fixierung des gebildeten Komplexes an einer Festphase der nichtgebundene Analyt durch Waschen der Festphase vor der Reaktion mit einem zweiten spezifischen markierten Rezeptor entfernt wird.

Sandwich-Assays können auch zum Nachweis von Antikörpern durchgeführt werden, beispielsweise als Brückentests, wobei ein für die nachzuweisenden Antikörper spezifisches immobilisiertes oder immobilisierbares Antigen und ein zweites markiertes Antigen verwendet wird. Derartige Brückentest- oder Doppelantigen-Sandwich-Bestimmungen sind beispielsweise in EP-A-0 280 211 beschrieben.

Auch Analytbestimmungen mit Hilfe des DSAP (Double Antibody Solid Phase)-Sandwichprinzips, bei welchem die beiden analytspezifischen Rezeptoren löslich sind und der Komplex mit dem Analyten mit einem weiteren Rezeptor an der Festphase fixiert wird, sind im obigen Sinne Einschritt-Tests und zeigen einen Hook-Effekt.

Zur Erkennung bzw. Vermeidung des Hook-Effekts wurden zahlreiche Versuche unternommen, z.B. die Durchführung des Assays in einem Zweischritt- oder Mehrschrittverfahren, wobei nach dem ersten Reaktionsschritt gewaschen wird (Hoffmann et al., Clin. Chem. 30 (1984), 1499). Nachteil dieses Verfahrens ist jedoch ein höherer Arbeitsaufwand, eine erhöhte Gesamtinkubationszeit und darüber hinaus oftmals ein Sensitivitätsverlust sowie unter Umständen keine komplette Beseitigung des Hook-Effekts.

Weiterhin wurde vorgeschlagen, mehrere Bestimmungen an einer einzigen Probe mit jeweils unterschiedlichen Verdünnungen durchzuführen. Auch dieses Verfahren führt jedoch zu einem erhöhten Zeit- und Arbeitsaufwand sowie zu erhöhten Kosten für den Anwender.

Hoffmann et al. (1984), supra, beschreiben zusätzlich ein Bestimmungsverfahren für einen Einschritt-Sandwichtest, bei dem die Reaktion des Analyten mit dem Rezeptor kinetisch verfolgt und die Werte in einem Computer gespeichert werden. Dabei können Meßergebnisse, die durch den Hook-Effekt hervorgerufen werden, von anderen Werten unterschieden werden. Dieses Verfahren besitzt den Nachteil, daß es äußerst aufwendige und teure Computeranalysen erfordert, um zu erkennen, ob bei der Bestimmung ein Hook-Effekt vorliegt.

Auch weitere Vorschläge, die zur Verringerung bzw. zur Vermeidung des Hook-Effekts gemacht wurden, führen zu erheblichen Nachteilen. So hat eine Verringerung des Probevolumens im allgemeinen einen erheblichen Sensitivitätsverlust zur Folge. Eine Erhöhung der Rezeptorkonzentration (Festphasenbindungsrezeptor oder/und markierter Rezeptor) führt oftmals zu deutlich erhöhten Kosten sowie bei Erhöhung der Konzentration des markierten Rezeptors zu Leerwertproblemen und damit verbunden zu einem Sensitivitätsverlust. Darüber hinaus hat die Erhöhung der Rezeptorkonzentration nur ein relativ geringes Potential zur Verringerung des Hook-Effekts.

Weiterhin wurde beispielsweise in US-A-4,595,661, US-A-4,743,542 oder EP-A-0 617 285 die Zugabe unmarkierter Antikörper oder Antigene zum Testreagenz vorgeschlagen. Auch diese führt jedoch in der Regel zu einem Sensitivitätsverlust und zu deutlich erhöhten Reagenzkosten.

EP-A-0 572 845 offenbart ein Verfahren zur immunchemischen Bestimmung eines Analyten in einer Probe mittels eines ersten spezifischen Bindungspartners, wobei der spezifische Bindungspartner an einem Träger immobilisiert ist und das Ausmaß der Bindung des Analyten an den spezifischen Bindungspartner mittels eines weiteren spezifischen Bindungspartners, der direkt oder indirekt eine Markierung trägt, bestimmt wird und wobei dem Verfahren zusätzlich ein Bindefaktor zugegeben wird, der mehr als eine bindungsfähige Stelle für den Analyten aufweist, keine Affinität zu dem immobilisierten spezifischen Bindungspartner aufweist und nicht markiert ist. Beispiele solcher Bindefaktoren sind mono- und polyklonale Antikörper, sowie Fragmente davon, Lectine bzw. Konjugate mehrerer Lectine oder Konjugate von Lectinen mit analytspezifischen Antikörpern oder deren Fragmente. Wenn der Analyt ein Antikörper ist, sind die als Bindefaktoren verwendeten Antikörper nicht homolog zu den Analytantikörpern und gegen die spezifische Immunglobulinklasse des Analytantikörpers gerichtet. Die Verwendung von Rheumafaktoren zur Bestimmung von Humanantikörpern wird weder offenbart noch nahegelegt.

Gemäß EP-A-0 572 845 findet die Ausbildung eines Immunkomplexes unabhängig davon statt, ob der Bindefaktor vorhanden ist oder nicht. Weiterhin wird betont, daß sich aufgrund einer dem Fachmann bekannten Rückreaktion der Komplex aus Analyt und spezifischen Rezeptor wieder auflösen und sich so der Nachweisreaktion entziehen kann, wodurch die Sensitivität stark eingeschränkt wird. Aus diesem Grund ist die diagnostische Leistungsfähigkeit des Tests eingeschränkt, wenn die Rückreaktionsrate zwischen immobilisiertem Rezeptor und nachzuweisendem Analyten sehr hoch ist. Eine solche Rückreaktionsrate ist vor allem im unteren Meßbereich, also bei niedrigen Konzentrationen des Analyten oder bei niederaffinen Anti-Analyt-Antikörpern von Bedeutung.

Die der vorliegenden Erfindung zugrundeliegende Aufgabe bestand somit darin, die oben genannten Nachteile des Standes der Technik zumindest weitgehend zu vermeiden. Diese Aufgabe wird dadurch gelöst, daß polyklonale oder/und monoklonale Rheumfaktoren oder Rheumafaktor-ähnliche Substanzen als Entstörungsreagenzien dem Testansatz zugegeben werden. Auf diese Weise können durch den Hook-Effekt bedingte Falschmessungen ohne zusätzlichen Aufwand an Zeit und Arbeit sowie ohne Sensitivitätsverlust verringert oder vollständig vermieden werden.

Ein Gegenstand der vorliegenden Erfindung ist somit ein Verfahren zur Bestimmung eines Analyten in einer Probe, welches dadurch gekennzeichnet ist, daß man zur Entstörung Rheumafaktoren oder Rheumafaktor-ähnliche Substanzen zusetzt. Vorzugsweise erfolgt die Bestimmung durch einen Sandwich-Assay, insbesondere durch einen Einschritt-Sandwich-Assay. Weiterhin ist bevorzugt, daß die Bestimmung durch ein heterogenes Nachweisverfahren, d.h. über eine Festphasenbindung des Analyten erfolgt.

Beim erfindungsgemäßen Verfahren steht die Vermeidung falsch-negativer Testresultate aufgrund des Hook-Effekts im Vordergrund. Eine möglicherweise hohe Rückreaktionsrate im Komplex aus Festphasen-gebundenen Analyten und Anti-Analytantikörper spielt keine wesentliche Rolle, da beim Hook-Effekt definitionsgemäß eine sehr hohe Analytkonzentration vorliegt und somit die Rückreaktionsrate nicht kritisch ist.

Rheumafaktoren sind Autoantikörper, die gegen körpereigenes Immunglobulin, beispielsweise IgG gerichtet sind. Meistens handelt es sich bei den Rheumafaktoren um Antikörper der Klasse IgM. Besonders bevorzugt sind humane Rheumafaktoren. Rheumafaktoren sind in der Literatur oftmals als Störsubstanzen für immunologische Nachweisverfahren beschrieben worden (vgl. z.B. EP-A-0 163 312; Henle et al., Clin. Exp. Immunol. 36 (1979), 415-422; Ho et al., J. Clin. Microbiol. 27 (1989), 952-958; Thorfason und Diderholm, J. Meth. Virol. (1982), 157-170; Espersen et al., Scand. J. Rheumathology, 75 (1988), 40-45; EP-A-0 292 810; DE-OS 42 02 923; PCT/EP 95/04307 und PCT/EP 95/04308). Angesichts dieses Standes der Technik war es in höchstem Maße überraschend, daß der Zusatz von Rheumafaktoren zu einer Verringerung oder sogar zu einer Beseitigung des Hook-Effekts führen kann.

Neben Rheumafaktoren können auch Rheumafaktor-ähnliche Substanzen eingesetzt werden, d.h. Entstörreagenzien, die gleiche oder ähnliche Eigenschaften aufweisen wie Rheumafaktoren. Insbesondere sind Rheumafaktor-ähnliche Substanzen in der Lage oligomere oder polymere bzw. vernetzte oder an den Analyten gebundene Immunglobuline besser zu binden als Immunglobuline in Monomerform. Unter Rheumafaktor-ähnlichen Substanzen sind dabei Bindefaktoren zu verstehen, die mehr als eine bioaffine Bindestelle für den Nachweisantikörper besitzen. Spezifische Beispiele solcher Rheumafaktor-ähnlicher Substanzen sind human-IgGbindende Peptide wie etwa Peptidfragmente von C1q, die im US-Patent 5,759,863 beschrieben sind, oder monoklonale IgM-Antikörper, die oligomeres Human-IgG von monomerem Human-IgG unterscheiden können. Solche Antikörper sind erhältlich durch Immunisierung von Versuchstieren mit vernetztem Human IgG und Gewinnung von monoklonalen IgM-Antikörpern mit hoher Affinität zu oligomerem Human-IgG und geringer Affinität zu monomerem Human-IgG.

Die Probe, in der der zu bestimmende Analyt vorliegt, ist vorzugsweise eine biologische Probe, z.B. eine aus einer Körperflüssigkeit stammende Probe wie etwa Blut, Serum, Plasma, Urin, Speichel, Sperma oder Cerebrospinalflüssigkeit oder eine Gewebe- oder Zellkulturprobe. Bevorzugt ist die Probe eine Blut-, Serum- oder Plasmaprobe, insbesondere eine humane Probe.

Das erfindungsgemäße Verfahren wird vorzugsweise als heterogener Sandwich-Assay ausgeführt und umfaßt die Schritte:
(a) Inkontaktbringen der Probe mit einer Festphase, einem ersten analytspezifischen Rezeptor, der an die Festphase gebunden oder mit der Festphase bindefähig ist, und einem zweiten analytspezifischen Rezeptor, der eine signalgebende Gruppe trägt oder mit einer signalgebenden Gruppe bindefähig ist und
(b) Nachweisen des Vorhandenseins oder/und der Menge des Analyten durch Bestimmung der signalgebenden Gruppe auf der Festphase.

Weiterhin bevorzugt umfaßt das erfindungsgemäße Verfahren eine immunologische Reaktion, d.h. eine Antigen-Antikörper-Wechselwirkung, die direkt oder indirekt zur Bestimmung des Analyten verwendet wird. Grundsätzlich kann das erfindungsgemäße Verfahren jedoch auch bei anderen Bestimmungreaktionen verwendet werden, die auf einer hochaffinen Wechselwirkung beruhen und bei denen die Gefahr des Auftretens eines Hook-Effekts besteht.

Der zu bestimmende Analyt kann jede in einer biologischen Probe vorliegende Substanz sein, beispielsweise ein in einer Körperflüssigkeit vorliegender Antikörper, insbesondere ein Autoantikörper, ein Tumorantikörper oder ein gegen ein Pathogen, z.B. einen Virus wie etwa HIV oder Hepatitisviren gerichteten Antikörper. Andererseits kann der Analyt auch ein Antigen, d.h. auch ein mit einem Antikörper bindefähige Substanz sein, beispielsweise ein Tumormarker wie etwa α-Fetoprotein oder Karzinogenembryonales Antigen oder ein Schwangerschaftsmarker wie etwa humanes Choriongonadotropin.

Vorzugsweise wird die erfindungsgemäße Entstörung durch Rheumafaktoren oder Rheumafaktor-ähnliche Substanzen bei der Bestimmung von Antikörpern, insbesondere von humanen Antikörpern, verwendet. Besonders bevorzugt ist dabei eine Bestimmung nach dem zuvor bereits beschriebenen Brückentestverfahren. Weiterhin bevorzugt ist auch das sog. indirekte Verfahren zum Nachweis von Antikörpern, wobei ein für den zu bestimmenden Antikörper spezifisches Antigen in immobilisierter oder immobilisierbarer Form eingesetzt wird und die Bindung des Analytantikörpers über einen gegen den Analytantikörper gerichteten Antikörper, der direkt oder indirekt markiert ist, nachgewiesen wird.

Sofern das Nachweisverfahren nicht als turbidimetrischer oder nephelometrischer Test durchgeführt wird, verwendet man zum Nachweis des Analyten üblicherweise eine signalgebende Gruppe, die aus im Stand der Technik bekannten Markierungen ausgewählt werden kann. Beispiele sind Radiomarkierungen, Enzyme, Farbstoffe, Fluoreszenzgruppen und Elektrochemilumineszenzgruppen. Besonders bevorzugt sind Elektrochemilumineszenzgruppen, z.B. lumineszierende Metallchelat-Markierungsgruppen wie etwa Rutheniumchelate, z.B. Ruthenium-(Bipyridyl)-₃-Komplexe, die in EP-A-0 178 450, EP-A-0 255 534, EP-A-0 580 979 und WO90/05301 beschrieben sind. Die signalgebende Gruppe kann entweder direkt an einen analytspezifischen Rezeptor (direkte Markierung) oder an einen weiteren Rezeptor, der mit dem analytspezifischen Rezeptor bindefähig ist (indirekte Markierung), gebunden sein.

Als Festphase können für das erfindungsgemäße Verfahren beispielsweise partikuläre Medien, insbesondere Mikrobeads wie etwa Latexpartikel und besonders bevorzugt magnetische Mikrobeads oder Oberflächen von Reaktionsgefäßen, insbesondere Mikrotiterplatten, Küvetten, Teströhrchen, Chips und Sensoren verwendet werden.

Bei Verwendung einer Festphase in einem heterogenen Nachweisverfahren enthält das Testreagenz einen festphasenseitigen analytspezifischen Rezeptor. Dieser festphasenseitige Rezeptor kann bereits vor dem Test an die Festphase gebunden werden, beispielsweise durch adsorptive oder kovalente Wechselwirkungen, bevorzugt jedoch durch spezifische hochaffine Wechselwirkungen, z.B. Streptavidin oder Avidin/Biotin oder Antikörper/Antigen-Wechselwirkungen. Andererseits kann der festphasenseitige Rezeptor auch in einer an die Festphase bindefähigen Form vorliegen, d.h. er wird erst während des Assays vorzugsweise über eine hochaffine Wechselwirkung an die Festphase gebunden. Besonders bevorzugt werden mit Streptavidin oder Avidin beschichtete Festphasen und biotinylierte festphasenseitige analytspezifische Rezeptoren verwendet. In diesem Zusammenhang ist anzumerken, daß die festphasenseitigen Rezeptoren sowohl direkt als auch indirekt über einen oder mehrere weitere Rezeptoren an die Festphase gebunden werden können.

Die Rheumafaktoren oder Rheumafaktor-ähnlichen Substanzen werden beim erfindungsgemäßen Verfahren dem Testansatz in einer ausreichenden Menge zugesetzt, um einerseits eine möglichst weitgehende Entstörung zu erreichen, d.h. den Hook-Effekt zu verringern, und andererseits die Durchführung der Nachweisreaktion nicht wesentlich zu beeinträchtigen. Hierzu können Rheumafaktoren vorzugsweise in einer Menge eingesetzt werden, so daß sie in einer Endkonzentration von 1 bis 1000 U/ml (gemäß WHO-Standard 64/2) im Testansatz vorliegen. In Abhängigkeit des Ausmaßes der Entstörung können bereits kleinste Konzentrationen an Rheumafaktoren oder Rheumafaktor-ähnlichen Substanzen wirksam sein. Die maximal einsetzbare Konzentration wird durch die Löslichkeit des Rheumafaktors oder der anderen Entstörsubstanzen bestimmt.

Die Rheumafaktoren können beim erfindungsgemäßen Verfahren in Form von polyklonalen oder/und monoklonalen Antikörpern, in aufgereinigter Form oder in Form von Serum, Plasma oder Fraktionen davon, eingesetzt werden. Gegebenenfalls kann man auch Antikörperfragmente verwenden.

In einer ersten Ausführungsform des erfindungsgemäßen Verfahrens werden die Rheumafaktoren oder Rheumafaktor-ähnliche Substanzen in löslicher Form direkt dem Testansatz zugegeben. In einer weiteren Ausführungsform werden die Rheumafaktoren oder Rheumafaktor-ähnlichen Substanzen - entsprechend dem festphasenseitigen analytspezifischen Rezeptor - gebunden an eine Festphase oder in einer mit der Festphase bindefähigen Form eingesetzt. So können beispielsweise bei Verwendung von mit Streptavidin oder Avidin beschichteten Festphasen biotinylierte Rheumafaktoren eingesetzt werden. In noch einer weiteren Ausführungsform wird zusätzlich ein für Rheumafaktoren oder Rheumafaktor-ähnliche Substanzen spezifischer festphasenseitiger Rezeptor verwendet, d.h. ein mit den Entstörungsreagenzien spezifisch bindefähiger Rezeptor, der selbst an die Festphase gebunden oder mit der Festphase bindefähig ist. Als Rheumafaktor-spezifischer Rezeptor kann beispielsweise ein Anti-IgM-Antikörper, z.B. ein Anti-Human-IgM-Antikörper verwendet werden. Auch in dieser Ausführungsform ist die Verwendung von mit Streptavidin oder Avidin beschichteten Festphasen und biotinylierten Rheumafaktoren oder biotinylierten Rheumafaktor-spezfischen Rezeptoren bzw. biotinylierten Rheumafaktor-ähnlichen Substanzen oder biotinylierten Rezeptoren dafür bevorzugt.

Ein weiterer Gegenstand der vorliegenden Erfindung ist die Verwendung von Rheumafaktoren oder Rheumafaktor-ähnliche Substanzen als Entstörungsreagenzien in einem Nachweisverfahren, insbesondere in einem diagostischen Nachweisverfahren zur Bestimmung eines Analyten in einer biologischen Probe, z.B. einer Körperflüssigkeit. Vorzugsweise werden Rheumafaktoren oder Rheumafaktor-ähnliche Substanzen zur Verringerung oder zur Vermeidung des Hook-Effekts insbesondere in einem immunologischen Verfahren eingesetzt.

Noch ein weiterer Gegenstand der Erfindung ist ein Reagenzienkit zur Bestimmung eines Analyten, der neben anderen Reagenzien als Entstörungsreagenz Rheumafaktoren oder Rheumafaktor-ähnliche Substanzen enthält. Vorzugsweise enthält der Reagenzienkit weiterhin eine Festphase, einen ersten analytspezifischen Rezeptor, der an die Festphase gebunden oder der mit der Festphase bindefähig ist und einen zweiten analytspezifischen Rezeptor, der eine signalgebende Gruppe trägt oder mit einer signalgebenden Gruppe bindefähig ist.

Weiterhin wird die Erfindung durch die nachfolgenden Beispiele und Abbildungen näher erläutert. Es zeigen:
- Abb. 1: das Ergebnis eines Nachweises von Anti-HBc-Antikörpern mit einer unterschiedlich verdünnten Probe in Abhängigkeit vom Zusatz von Rheumafaktoren,
- Abb. 2: das Ergebnis des Nachweises von Anti-HBs-Antikörpern in verschiedenen Probenverdünnungen in Abhängigkeit vom Zusatz von Rheumafaktoren,

### Beispiele

### 1. Nachweis von Anti-Hepatitis B Core-(HBc)-Antikörpern durch Elektro chemilumineszenz

Der Test wurde als Doppelantigen-Sandwich-Assay durchgeführt und beruht auf der Verwendung eines biotinylierten HBc-Antigens und eines mit einem elektrochemilumineszenzfähigen Rutheniumkomplex-markierten HBc-Antigens.

Die Biotinylierung des Antigens kann nach dem Fachmann bekannten Methoden erfolgen. Die Herstellung von Ruthenium-markierten Antigenen kann beispielsweise nach der in WO 96/03651 beschriebenen Methode erfolgen.

Ein für Anti-HBc-Antikörper-positives Serum mit einem Gehalt von ca. 10 000 U/ml wurde in verschiedenen Verdünnungsstufen mit bzw. ohne Zusatz von Rheumafaktoren und Anti-Human-IgM-Antikörper-Biotinkonjugaten sowie mit Streptavidin (SA) beschichteten Magnetpartikeln vermessen.

Zur Messung wurde das Gerät Elecsys® 2010 (Hersteller Boehringer Mannheim/Hitachi) entsprechend der Bedienungsanleitung mit der Programmierung "Testprotokoll 2" verwendet. Die Testreagenzien wurden wie folgt eingesetzt:
- Volumina: 10 µl Probe, 75 µl Reagenz 1, 75 µl Reagenz 2, 40 µl Streptavidin-Beads
- Inkubationszeiten: 9 min Reagenz 1 und Reagenz 2 mit Probe, weitere 9 min mit SA-Beads
- Reagenz 1: 100 mM Na-Phosphatpuffer, pH 7,4, Rinderserumbestandteile, Detergenz, Konservierungsmittel und biotinyliertes HBcAg (1400 ng/ml)
- Reagenz 2: 100 mM Na-Phosphatpuffer, pH 7,4, Rinderserumbestandteile, Detergenz, Konservierungsmittel und ruthenyliertes HBcAg (700 ng/ml)

Biotinyliertes und ruthenyliertes HBcAg werden zusammen mit der Probe 9 min inkubiert. Nach Zugabe von Streptavidin-beschichteten Beads wird weitere 9 min inkubiert, bevor die Elektrochemilumineszenz bestimmt wird.

In dem vorliegenden Beispiel enthält Reagenz 1 zusätzlich 0 oder 200 U/ml Rheumafaktor sowie Reagenz 2 zusätzlich 0 oder 700 ng/ml biotinylierte monoklonale anti-h-IgM-Antikörper.

Die Endkonzentration der Rheumafaktoren im Testansatz beträgt 74 U/ml.

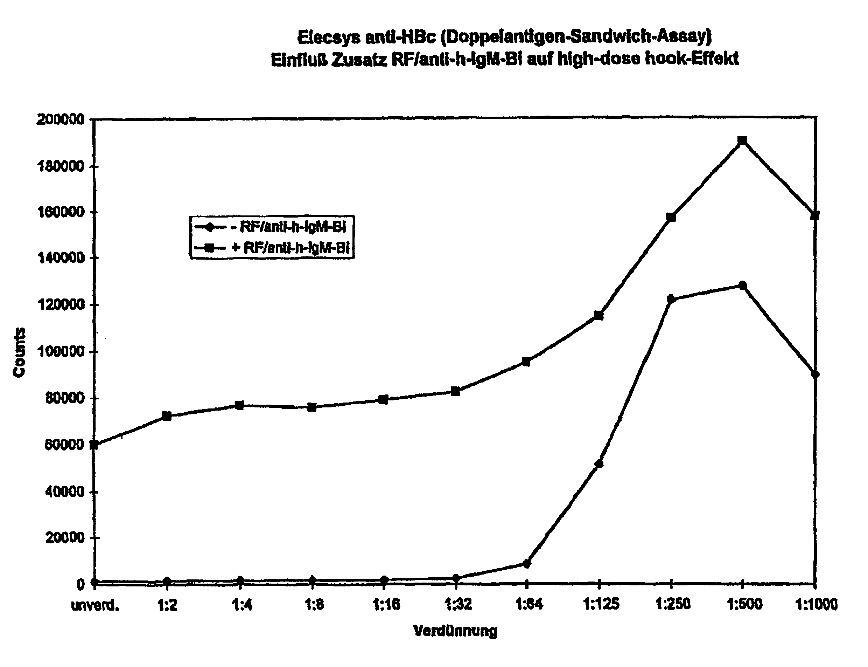

Das Ergebnis des Versuchs ist in Abb. 1 sowie in der nachfolgenden Tabelle 1 gezeigt.

**Tabelle 1**

| **Probenverdünnung** | **-RF/anti-h-IgM-Bi** **(Counts)** | + **RF/anti-h-IgM-Bi** **(Counts)** |
|---|---|---|
| unverd. | 1287 | 59881 |
| 1:2 | 1503 | 72196 |
| 1:4 | 1662 | 76729 |
| 1:8 | 1716 | 75870 |
| 1:16 | 1794 | 78988 |
| 1:32 | 2371 | 82210 |
| 1:64 | 8453 | 94805 |
| 1:125 | 51329 | 114814 |
| 1:250 | 121566 | 156925 |
| 1:500 | 127260 | 190038 |
| 1:1000 | 89452 | 157607 |

Während ohne Zusatz von Rheumafaktoren und biotinyliertem Anti-IgM das positive Anti-HBc-Serum erst ab einer Vorverdünnung von 1:64 eine Differenzierung von positiv zu negativ möglich ist, kann bei Zusatz von Rheumafaktoren und biotinyliertem Anti-IgM bereits in der unverdünnten Probe ein positiver Befund (mehr als 59000 Counts) erhalten werden.

### 2. Nachweis von Anti-Hepatitis B Surface (HBs)-Antikörpern durch Elektrochemilumineszenz

Auch dieser Test erfolgte als Doppelantigen-Sandwich-Assay unter Verwendung eines biotinylierten HBs-Antigens und eines Ruthenium-markierten HBs-Antigens am Elecsys® 2010 Gerät mit der Programmierung "Testprotokoll 2".

Ein hochtitiriges positives HBs-Serum (ca. 400 000 IU/L) wurde in verschiedenen Verdünnungsstufen mit bzw. ohne Zusatz von Rheumafaktoren und Anti-Human-IgM-Biotinkonjugat vermessen. Die Reagenzien wurden wie folgt eingesetzt:
- Volumina: 40 µl Probe, 65 µl Reagenz 1, 60 µl Reagenz 2, 35 µl Streptavidin-Beads
- Inkubationszeiten: 9 min Reagenz 1 und Reagenz 2 mit Probe, weitere 9 min mit SA-Beads
- Reagenz 1: 80 mM MES-Puffer, pH 6,5 Rinderserumbestandteile, Detergenz, Konservierungsmittel und biotinyliertes HBsAg (800 ng/ml)
- Reagenz 2: 80 mM MES-Puffer, pH 6,5, Rinderserumbestandteile, Detergenz, Konservierungsmittel und ruthenyliertes HBsAg (500 ng/ml)

Biotinyliertes und ruthenyliertes HBsAg werden zusammen mit der Probe 9 min inkubiert. Nach Zugabe von Streptavidin-beschichteten Beads wird weitere 9 min inkubiert, bevor die Elektrochemilumineszenz bestimmt wird.

In dem vorliegenden Beispiel enthält Reagenz 1 zusätzlich 0 oder 200 U/ml Rheumafaktor sowie Reagenz 2 zusätzlich 0 oder 700 ng/ml biotinylierte monoklonale anti-h-IgM-Antikörper.

Die Endkonzentration der Rheumafaktoren im Testansatz war 65 U/ml.

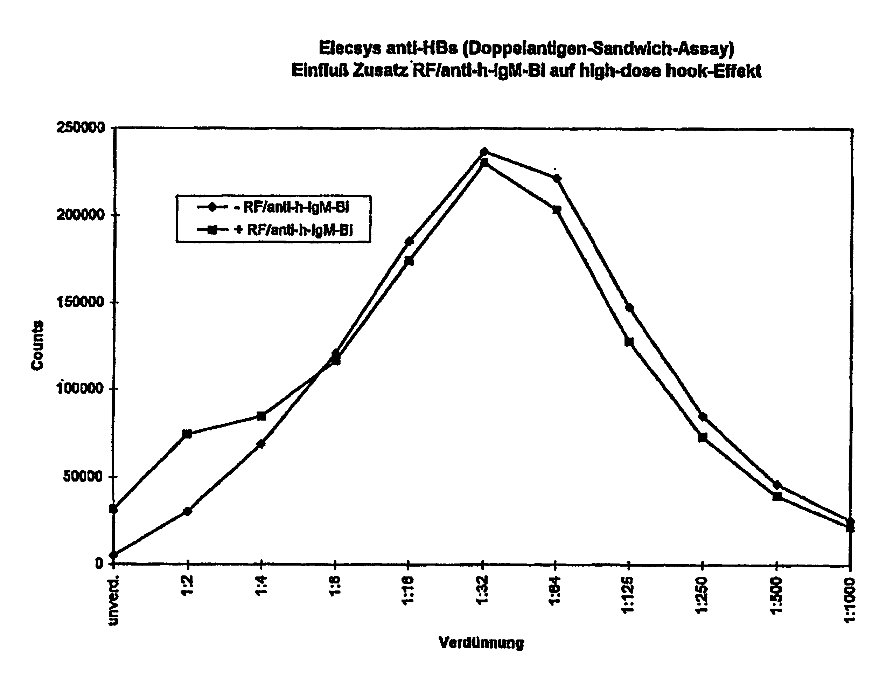

Das Ergebnis des Versuchs ist in Tabelle 2 und Abb. 2 gezeigt.

**Tabelle 2**

| **Probenverdünnung** | **anti-HBs-Konz.** **(IU/L)** | **-RF/anti-h-IgM-Bi** **(Counts)** | **+ RF/anti-h-IgM-Bi** **(Counts)** |
|---|---|---|---|
| unverd. | 386000 | 4880 | 31483 |
| 1:2 | 193000 | 30097 | 74587 |
| 1:4 | 96500 | 69192 | 84998 |
| 1:8 | 48250 | 120823 | 116538 |
| 1:16 | 24125 | 185021 | 173833 |
| 1:32 | 12063 | 236592 | 230159 |
| 1:64 | 6021 | 221533 | 203239 |
| 1:125 | 3016 | 147461 | 127618 |
| 1:250 | 1508 | 85302 | 73405 |
| 1:500 | 705 | 46226 | 39386 |
| 1:1000 | 386 | 25662 | 22039 |

Aus den Ergebnissen ist ersichtlich, daß durch Zusatz von Rheumafaktoren und biotinyliertem Anti-IgM das Auftreten des Hook-Effekts in einen höheren Konzentrationsbereich ohne Verlust der Testsensitivität verschoben werden kann. Bei unverdünnten oder nur gering verdünnten Proben (1:2 oder 1:4) wird durch den Zusatz von Rheumafaktoren ein signifikanter Sensitivitätsgewinn erzielt.

### 3. Nachweis von Anti-HIV-Antikörpern durch Elektrochemilumineszenz

Der Test wurde entsprechend dem HIV-Kombitest gemäß der deutschen Patentanmeldung DE 197 27 943.0 durchgeführt. Es handelt sich hierbei um einen kombinierten HIV-Antigen-/Antikörper-Nachweis. Als festphasengebundene Rezeptoren werden biotinylierte monoklonale Anti-p24-Antikörper (R1) und verschiedene biotinylierte HIV-Antigene (R3 und R5) eingesetzt. Als markierte, zur Detektion verwendete, ruthenylierte Rezeptoren werden ruthenylierte monoklonale Anti-p24-Antikörper (R2) und ruthenylierte HIV-Antigene (R4) und (R6) eingesetzt:
- R1: biotinylierte monoklonale anti-p24-lgG Antikörper
- R2: ruthenylierte monoklonale anti-p24-IgG Antikörper
- R3: biotinylierte gp36-, gp41-Peptide und Polyhaptene entsprechend den im Enzymun®-Test Anti-HIV1+2+SubO, Best-Nr. 1557319, Boehringer Mannheim, Deutschland, verwendeten Peptiden
- R4: ruthenylierte gp36-, gp41-Peptide und Polyhaptene; die Sequenzen entsprechen den im Enzymun®-Test Anti-HIV1 + 2 + SubO, Best.-Nr. 1557319, Boehringer Mannheim, Deutschland, verwendeten Peptiden; sie sind jedoch ruthenyliert und nicht mit Digoxigenin markiert
- R5: biotinyliertes RT-Antigen hergestellt aus RT (Reverse Transkriptase), Best-Nr. 1465333, Boehringer Mannheim, Deutschland
- R6: ruthenyliertes RT-Antigen hergestellt aus RT, Best.-Nr. 1465333, Boehringer Mannheim, Deutschland

Ruthenium-markierte HIV-Antigene und deren Herstellung sind beispielsweise in der WO 96/03651 beschrieben. Die Biotinylierung von Antigenen erfolgt nach dem Fachmann bekannten Methoden. Die Herstellung von Polyhaptenen ist in der WO 96/03652 erläutert.

Hochtitrigen anti-HIV-positiv Proben wurden in zwei Verdünnungsstufen mit bzw. ohne Zusatz von Rheumafaktoren (Konzentrationen 20, 70, 200 U/ml in Reagenz 1) vermessen.

Die Versuchsdurchführung erfolgte an dem Gerät Elecsys® 2010 entsprechend Bedienungsanleitung mit der Programmierung "Testprotokoll 2". Es wurden Testreagenzien wie folgt verwendet:
- Volumina: 50 µl Probe, 50 µl Reagenz 1, 50 µl Reagenz 2, 50 µl Streptavidin-Beads
- Inkubationszeiten: 9 min Reagenz 1 und Reagenz 2 mit Probe, weitere 9 min mit Beads
- Reagenz 1: Trispuffer pH 8,0, Rinderserumbestandteile, Detergenz, Konservierungsmittel und biotinylierte Antigene bzw. Antikörper (R1, R3, R5)
- Reagenz 2: Trispuffer pH 8,0, Rinderserumbestandteile, Detergenz, Konservierungsmittel und ruthenylierte Antigene bzw. Antikörper (R2, R4, R6)

Die Rezeptoren R1 bis R6 (Antikörper, Polyhaptene und RT jeweils 100-2000 ng/ml, Peptide 10-100 ng/ml) in den Reagenzien 1 und 2 werden zusammen mit der Probe 9 min inkubiert. Nach Zugabe von Streptavidin-beschichteten Beads wird weitere 9 min inkubiert, bevor die Elektrochemilumineszenz bestimmt wird.

In dem vorliegenden Beispiel enthält Reagenz 1 zusätzlich 0, 20, 70 oder 200 U/ml Rheumafaktor, indem die entsprechende Menge Humanserum (Rheumaserum) zugesetzt wurde (Endkonzentration der Rheumafaktoren im Testansatz: 0; 5; 17,5 bzw. 50 U/ml).

Das Ergebnis des Versuchs ist in Tabelle 3 dargestellt.

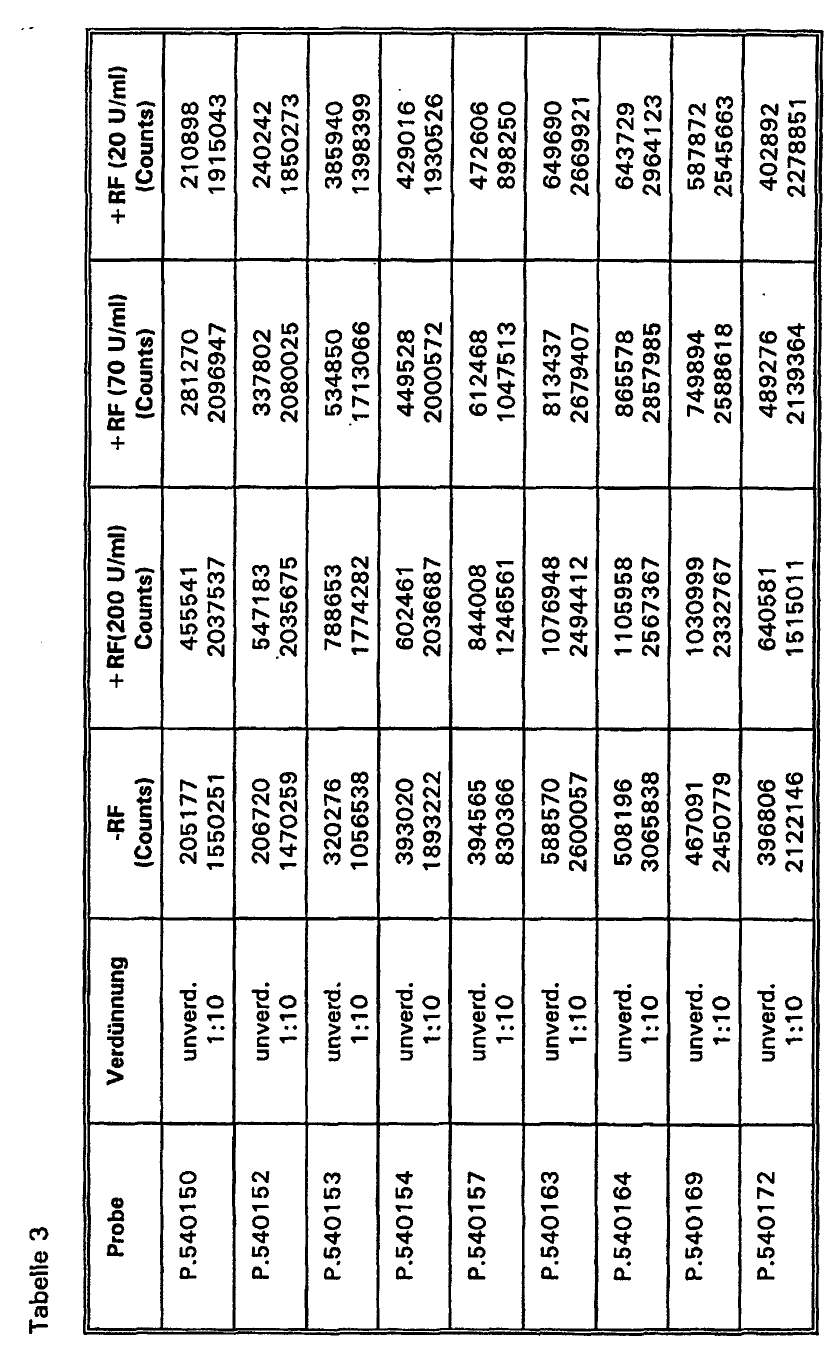

Aus den Ergebnissen ist ersichtlich, daß die Anwesenheit von Rheumafaktoren den Hook-Effekt deutlich verringert. Während bei verdünnten Proben nur ein geringer oder sogar gar kein Effekt zu sehen ist, wird bei den unverdünnten Proben das Meßsignal stark erhöht und der Hook-Effekt somit deutlich abgeschwächt.

## Patentansprüche

1. Verfahren zur Bestimmung eines Analyten in einer Probe,
**dadurch gekennzeichnet,**
**daß** man zur Verringerung oder Vermeidung eines Hook-Effektes als Entstärreagenz Rheumafaktoren oder Rheumafaktor-ähnliche Substanzen zusetzt.

2. Verfahren nach Anspruch 1,
**dadurch gekennzeichnet,**
**daß** es die Schritte umfasst:
(a) Inkontaktbringen der Probe mit einer Festphase, einem ersten analytspezifischen Rezeptor, der an die Festphase gebunden oder mit der Festphase bindefähig ist, und einem zweiten analytspezifischen Rezeptor, der eine signalgebende Gruppe trägt oder mit einer signalgebenden Gruppe bindefähig ist und
(b) Nachweisen des Vorhandenseins oder/und der Menge des Analyten durch Bestimmung der signalgebenden Gruppe auf der Festphase.

3. Verfahren nach Anspruch 1 oder 2,
**dadurch gekennzeichnet,**
**daß** die Rheumafaktoren oder Rheumafaktor-ähnlichen Substanzen in einer Endkonzentration von 1 bis 1000 IU/ml Testansatz vorliegen.

4. Verfahren nach einem der vorhergehenden Ansprüche,
**dadurch gekennzeichnet,**
**daß** zusätzlich ein für die Rheumafaktoren oder Rheumafaktor-ähnlichen Substanzen spezifischer Rezeptor verwendet wird, der an eine Festphase gebunden oder mit einer Festphase bindefähig ist.

5. Verwendung von Rheumafaktoren oder Rheumafaktor-ähnlichen Substanzen als Enstörungsreagenzien in einem Nachweisverfahren.

6. Verwendung nach Anspruch 5 zur Verringerung oder Vermeidung des Hook-Effekts.

7. Verfahren zur Entstörung von Immunoassays durch den Zusatz von Rheumafaktoren oder Rheumafaktor-ähnlichen Substanzen.

8. Reagenzienkit zur Bestimmung eines Analyten,
**dadurch gekennzeichnet,**
**daß** er neben anderen Reagenzien als Entstörungsreagenz Rheumafaktoren oder Rheumafaktor-ähnliche Substanzen enthält.

## Claims

1. Method for the determination of an analyte in a sample,
**wherein**
rheumatoid factors or rheumatoid-factor-like substances are added as an interference-reducing reagent or avoid a hook effect.

2. Method as claimed in claim 1,
**wherein**
it comprises the steps:
(a) contacting the sample with a solid phase, a first analyte-specific receptor which is bound to the solid phase or is capable of binding to the solid phase, and a second analyte-specific receptor which carries a signal-generating group or is capable of binding to a signal-generating group and
(b) detecting the presence or/and amount of the analyte by determining the signal-generating group on the solid phase.

3. Method as claimed in claim 1 or 2,
**wherein**
the rheumatoid factors or rheumatoid-factor-like substances are present at a final concentration of 1 to 1000 IU/ml test mixture.

4. Method as claimed in one of the previous claims,
**wherein**
a specific receptor for the rheumatoid factors or rheumatoid-factor-like substances is additionally used which is bound to a solid phase or is capable of binding to a solid phase.

5. Use of rheumatoid factors or rheumatoid-factor-like substances as interference-reducing reagents in a detection method.

6. Use as claimed in claim 5 to reduce or avoid the hook effect.

7. Method for reducing interference in immunoassays by adding rheumatoid factors or rheumatoid-factor-like substances.

8. Reagent kit for the determination of an analyte,
**wherein**
in addition to other reagents it contains rheumatoid factors or rheumatoid-factor-like substances as an interference-reducing reagent.

## Revendications

1. Procédé de détermination d'un analyte dans un échantillon **caractérisé en ce que** l'on ajoute, comme réactif d'élimination des perturbations, des facteurs. rhumatoïdes ou des substances analogues à des facteurs rhumatoïdes pour atténuer ou éviter un effet Hook.

2. Procédé selon la revendication 1 **caractérisé en ce qu'**il comprend les étapes suivantes :
(a) mise en contact de l'échantillon avec une phase solide, un premier récepteur spécifique de l'analyte, qui est lié à la phase solide ou capable de se lier à la phase solide, et un second récepteur spécifique de l'analyte, qui porte un groupe donnant un signal ou qui est capable de se lier à un groupe donnant un signal et
(b) mise en évidence de la présence et/ou de la quantité de l'analyte par détermination du groupe donnant un signal sur la phase solide.

3. Procédé selon la revendication 1 ou 2 **caractérisé en ce que** les facteurs rhumatoïdes ou les substances analogues à des facteurs rhumatoïdes sont présents en une concentration finale de 1 à 1000 UI/ml de mélange de test.

4. Procédé selon l'une des revendications précédentes **caractérisé en ce que** l'on utilise en outre un récepteur spécifique des facteurs rhumatoïdes ou des substances analogues à des facteurs rhumatoïdes qui est lié à une phase solide ou qui est capable de se lier à une phase solide.

5. Utilisation de facteurs rhumatoïdes ou de substances analogues à des facteurs rhumatoïdes comme réactifs d'élimination des perturbations dans un procédé de mise en évidence.

6. Utilisation selon la revendication 5 pour atténuer ou éviter l'effet Hook.

7. Procédé pour éliminer les perturbations d'immunodosages par addition de facteurs rhumatoïdes ou de substances analogues à des facteurs rhumatoïdes.

8. Trousse de réactifs pour la détermination d'un analyte **caractérisée en ce qu'**elle contient, outre d'autres réactifs, des facteurs rhumatoïdes ou des substances analogues à des facteurs rhumatoïdes comme réactif d'élimination des perturbations.
